# EUROPEAN PATENT APPLICATION

(11) **EP 1 285 642 A1**
(43) Date of publication of application: **26.02.2003**
(21) Application number: 02025743.2
(22) Date of filing: 07.08.2001
(51) Int. Cl.: A61F 9/007, A61M 1/00

(54) **Apparatus and software for controlling an intraoperative temperature**

(30) Priority: 29.08.2000 US 651541
(62) Divisional of application: 01959592.5
(71) Applicant: Alcon Manufacturing Ltd., Fort Worth, Texas 76134-2099 (US)
(72) Inventor: Morgan, Michael D., Costa Mesa, California 92627 (US); Boukhny, Mikhail, Laguna Niguel, California 92677 (US)
(74) Representative: Hanna, Peter William Derek

(57) **Abstract**

Apparatus and software for controlling an intraoperative temperature at a wound site is described. The apparatus comprises a control system having,
i) a control console (14,114,214,314,414,514,714) having a control module including the central processing unit (16,116,216,316,416,516,716);
ii) a handpiece power supply (20,120,220,320,420,520,720) and
iii) a means for measuring infusion fluid flow (22,122,222,322,422,522,722).
In order to control an intraoperative temperature at a wound site means are provided for;
a. selecting a desired intraoperative wound site temperature;
b. providing infusion fluid flow information to the control module;
c. providing handpiece power information to the control module from the handpiece power supply;
d. calculating a current temperature at the would site based on current and previous values of infusion fluid flow and handpiece power;
e. comparing the calculated current temperature at the wound site with the selected desired intraoperative wound site temperature; and
f. varying the operation of the handpiece power supply based on the comparison between the calculated current temperature at the wound site with the selected desired intraoperative wound site temperature.

## Description

This invention relates generally to the field of cataract surgery and more particularly to an infusion control system for a phacoemulsification handpiece.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical technique called phacoemulsification. During this procedure, a thin phacoemulsification cutting tip is inserted into the diseased lens and vibrated ultrasonically. The vibrating cutting tip liquefies or emulsifies the lens so that the lens may be aspirated out of the eye. The diseased lens, once removed, is replaced by an artificial lens.

A typical ultrasonic surgical device suitable for ophthalmic procedures consists of an ultrasonically driven handpiece, an attached cutting tip, and irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece by flexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve. Ultrasonic handpieces and cutting tips are more fully described in U.S. Pat. Nos. 3,589,363; 4,223,676; 4,246,902; 4,493,694; 4,515,583; 4,589,415; 4,609,368; 4,869,715; 4,922,902; 4,989,583; 5,154,694 and 5,359,996.

In use, the ends of the cutting tip and irrigating sleeve are inserted into a small incision of predetermined width in the cornea, sclera, or other location. The cutting tip is ultrasonically vibrated along its longitudinal axis within the irrigating sleeve by the crystal-driven ultrasonic horn, thereby emulsifying the selected tissue in situ. The hollow bore of the cutting tip communicates with the bore in the horn that in turn communicates with the aspiration line from the handpiece to the console. A reduced pressure or vacuum source in the console draws or aspirates the emulsified tissue from the eye through the open end of the cutting tip, the cutting tip and horn bores and the aspiration line and into a collection device. The aspiration of emulsified tissue is aided by a saline flushing solution or irrigant that is injected into the surgical site through the small annular gap between the inside surface of the irrigating sleeve and the cutting tip.

The preferred surgical technique is to make the incision into the anterior chamber of the eye as small as possible in order to reduce the risk of induced astigmatism. These small incisions result in very tight wounds that squeeze the irrigating sleeve tightly against the vibrating tip. Friction between the irrigating sleeve and the vibrating tip generates heat, but the risk of the tip overheating and causing a burn to the tissue is reduces by the cooling effect of the aspirated fluid flowing inside the tip. When the tip becomes occluded with tissue, this aspiration flow can be reduced or eliminated, allowing the tip to heat up.

Prior art devices have used sensors that detect large rises in aspiration vacuum, and predict occlusions based on vacuum rise. Based on this sensed occlusion, power to the handpiece may be reduced and/or irrigation and aspiration flows can be increased. See U.S. Patent Nos. 5,591,127, 5,700,240 and 5,766,146 (Barwick, Jr., et al.). Increased vacuum levels in the aspiration line, however, do not necessarily indicate that the flow of cooling fluid around the tip has been cut off. Even with the tightest incisions, some irrigating fluid will leak out between the wound and the outside of the irrigating sleeve. The wound leakage also provides additional cooling flow to the incision site, and measuring rises in aspiration vacuum alone does not necessarily indicate that a potential for a corneal burn exists. Therefore, power to the handpiece may be interrupted prematurely.

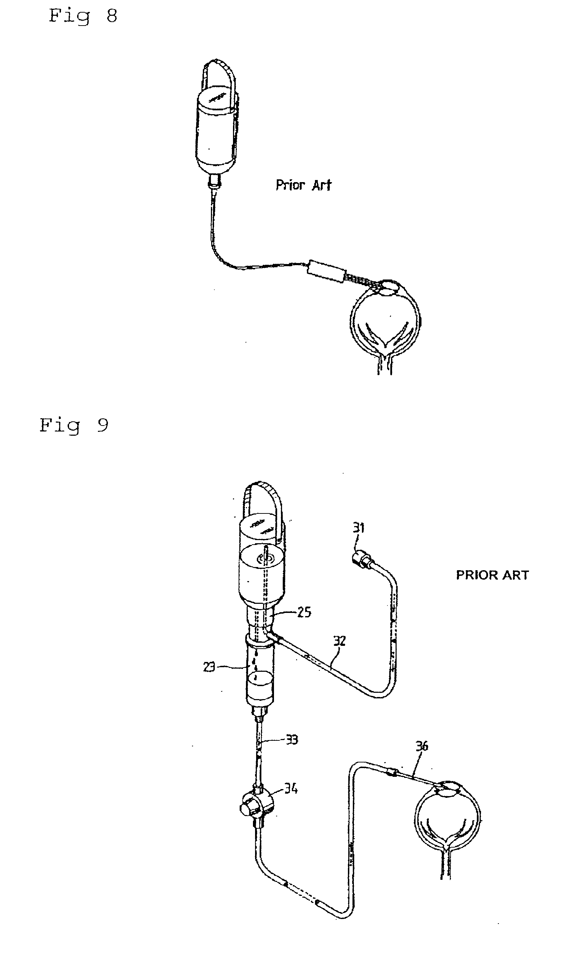

Prior art devices have also used gravity fed methods or pressurized gas sources for controlling surgical infusion pressure and flow. Gravity feed infusion methods, such as those illustrated in FIG. 8, provide a pressure and flow based on the height of a column of liquid. The higher the column, the greater the pressure and flow. The lower the column, the lower the pressure and flow. The surgeon controls the column height by raising or lowering the infusion bottle. Pressurized gas sources, such as those illustrated in FIG. 9, control the infusion pressure by increasing or decreasing the pressure inside the infusion bottle. The bottle is suspended at a constant height and a gas pressure pump is connected to the bottle. See U.S. Patent Nos. 4, 813,927, 4,900,301, 5,032,111 and 5,047,009(Morris, et al.), the entire contents of which being incorporated herein by reference. Gravity feed methods have limitations on pressure response rates due to the requirements of raising and lowering the infusion bottle. Pressurized gas methods improve on the response rates but require cumbersome venting snorkel devices that complicate the surgical setup. Both methods require filtering of air or gas into the bottle to prevent contamination which is added cost and complexity

Therefore, a need continues to exist for an infusion source for a surgical applications that utilizes a better method of infusion pressure and flow.

Our European Patent No. 1,062,958 describes a method of operating a central processing unit in a control system for a surgical device having a handpiece adapted to deliver irrigation fluid from a source to, and draw aspiration fluid from, an operative site, said control system comprising a control console having a control module including the central processing unit, and an irrigation flow sensor in the control console or in the handpiece, capable of providing irrigation fluid flow data to the central processing unit. Software performs the steps of;
setting a predetermined infusion fluid flow rate in the control module,
monitoring current actual infusion fluid flow rate data measured by the irrigation flow sensor, or as calculated by the central processing unit,
comparing the current actual or calculated infusion fluid flow rate with the predetermined infusion fluid flow rate, and if below the predetermined infusion fluid flow rate,
using the fluid flow data to control the operating parameters of the surgical device with software commands to vary any one or more of;
- the operation of an aspiration pump,
- the output of the handpiece power supply,
- the irrigation fluid flow through a valve,
- the output of a pressurizing source for the irrigation fluid,
or to provide audible tones.

Reference is also made to our European Patent Application No. 01959592.5 from which the present application is divided.

### Brief Summary of the Invention

The present invention improves upon the prior art by providing apparatus and software for controlling an intraoperative temperature at a wound site, in accordance with claims which follow, which includes a surgical infusion system having a variety of infusion fluid pressure sensors. The system may also use a collapsible infusion container. The information provided by the infusion fluid pressure sensors allows the users to predict and control intraoperative wound site temperature.

Accordingly, one objective of the present invention is to provide a surgical console control system. Another objective is to provide a method of operating a surgical console control system having infusion fluid pressure sensing capability.

Another objective of the present invention is to provide software for operating a surgical console control system that provides more accurate control of intraoperative wound site temperature.

Another objective of the present invention is to provide faster and more accurate control of infusion pressure and flow.

These and other advantages and objectives of the present invention will become apparent from the detailed description and claims that follow.

### Brief Description of the Drawings

FIG. 1 is a block diagram of a first embodiment of a control system that can be used with the present invention.
FIG. 2 is a block diagram of a second embodiment of a control system that can be used with the present invention.
FIG. 3 is a block diagram of a third embodiment of a control system that can be used with the present invention showing the flow sensor in the instrument and pressurized infusion control of the infusion fluid source.
FIG. 4 is a block diagram of a fourth embodiment of a control system that can be used with the present invention showing the flow sensor in the handpiece and pressurized infusion control of the infusion fluid source.
FIG. 5 is a block diagram of a fifth embodiment of a control system that can be used with the present invention showing the flow sensor in the instrument and measuring air flow of the pressurized infusion fluid source to calculate infusion fluid flow.
FIG. 6 is a block diagram of a sixth embodiment of a control system that can be used with the present invention showing the pressurized infusion fluid source as a compressed compliant bag and the infusion fluid flow calculated from the rate of infusion fluid source compression.
FIG. 7 is a flow chart illustrating the operation of an infusion flow control mode that can be used with the present invention.
FIG. 8 is an illustration of a prior art gravity fed infusion method.
FIG. 9 is an illustration of a prior art pressurized infusion method.
FIG. 10 is a block diagram of a one embodiment of the compliant container that can be used with the present invention being compressed between rollers.
FIG. 11 is a block diagram of another embodiment of the compliant container that can be used with the present invention being compressed by a pressure plate.
FIG. 12 is a block diagram of a yet another embodiment of a control system that can be used with the present invention having an irrigation line pressure sensor and pressurized infusion control of the infusion fluid source.

### Detailed Description of the Invention

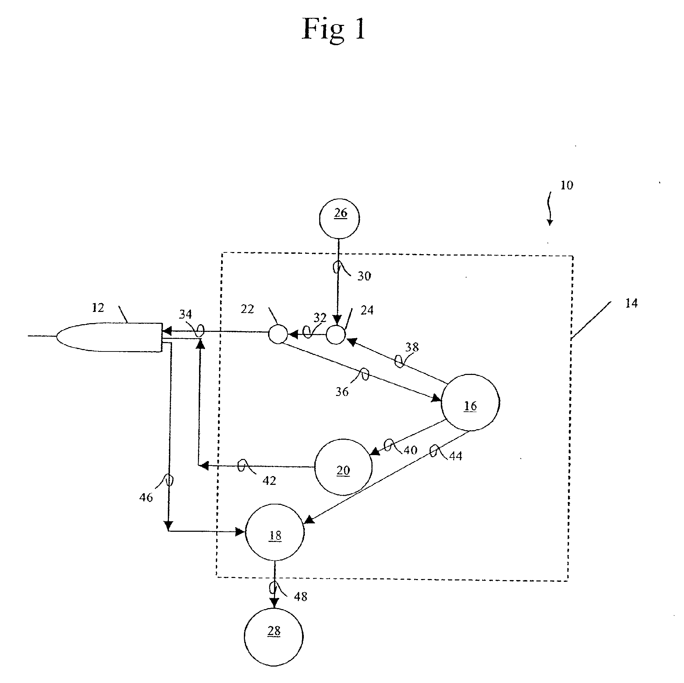

As seen in FIG. 1, control system 10 for use in operating handpiece 12 includes control console 14. Control console 14 generally includes control module or CPU 16, aspiration pump 18, handpiece power supply 20, irrigation flow sensor 22 and valve 24. Console 14 may be any commercially available surgical control console such as the ACCURUS® or LEGACY® SERIES TWENTY THOUSAND® surgical systems available from Alcon Laboratories, Inc., Fort Worth, Texas. CPU 16 may be any suitable microprocessor, micro controller, computer or digital logic controller. Pump 18 may be any suitable pump, such as a peristaltic, scroll, diaphragm or venturi pump. Power supply 20 may be any suitable ultrasound driver, such as incorporated in the ACCURUS® or LEGACY® SERIES TWENTY THOUSAND® surgical systems available from Alcon Laboratories, Inc., Fort Worth, Texas. Sensor 22 may be any commercially available flow sensor, such as Models Nos. T101D or T201D available from Transonic Systems, Inc., Ithaca, New York. Valve 24 may be any suitable valve such as a solenoid-activated pinch valve. Infusion source 26 may be any commercially available irrigation solution.

In use, sensor 22 is connected to handpiece 12 and infusion fluid source 26 through irrigation lines 30, 32 and 34. Sensor 22 measures the flow of irrigation fluid from source 26 to handpiece 12 and supplies this information to CPU 16 through cable 36. The irrigation fluid flow data may be used by CPU 16 to control the operating parameters of console 14 using software commands that are well-known in the art. For example, CPU 16, through cable 38, may open and close valve 24 so as to vary the amount of irrigation fluid reaching handpiece 12 from source 26. CPU 16 may also, through cable 40, vary the output of power supply 20 being sent to handpiece 12 though power cable 42. CPU 16 may also use data supplied by sensor 22 to vary the operation of pump 18, which aspirates fluid from handpiece 12 through line 46 and into collection container 28 through line 48.

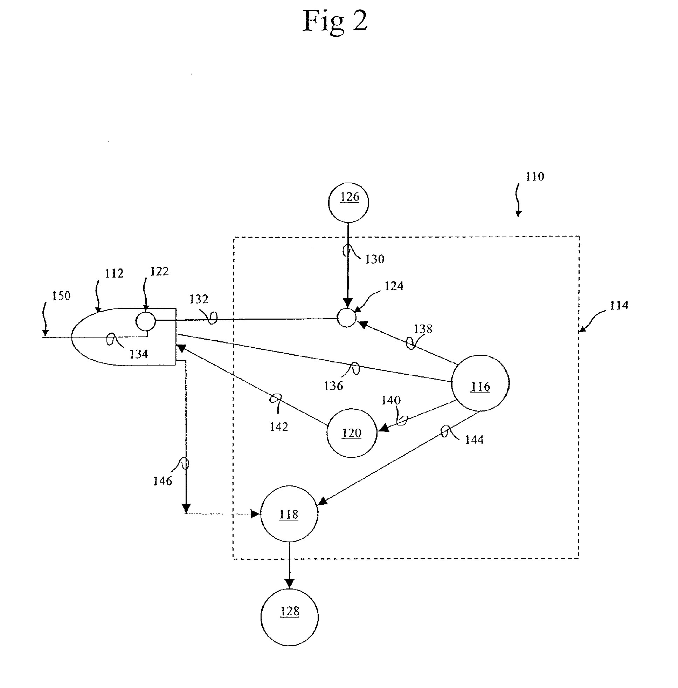

As seen in FIG. 2, control system 110 for use in operating handpiece 112 includes control console 114. Control console 114 generally includes control module or CPU 116, aspiration pump 118, handpiece power supply 120 and valve 124. Flow sensor 122 is contained within handpiece 112.

In use, tip 150 is connected to fluid source 126 through sensor 122 through irrigation lines 130, 132 and 134. Sensor 122 measures the flow of irrigation fluid from source 126 to tip 150 and supplies this information to CPU 116 through cable 136. CPU 116, through cable 138, may open and close valve 124 so as to vary the amount of irrigation fluid reaching tip 150 from source 126. CPU 116 may also, through cable 140, vary the output of power supply 120 being sent to handpiece 112 though power cable 142. CPU 116 may also use data supplied by sensor 122 to vary the operation of pump 118, which aspirates fluid from handpiece 112 through line 146 and into collection container 128 through line 148. CPU 116 may also use data supplied by sensor 122 and the applied output of power supply 120 to provide audible tones to the user.

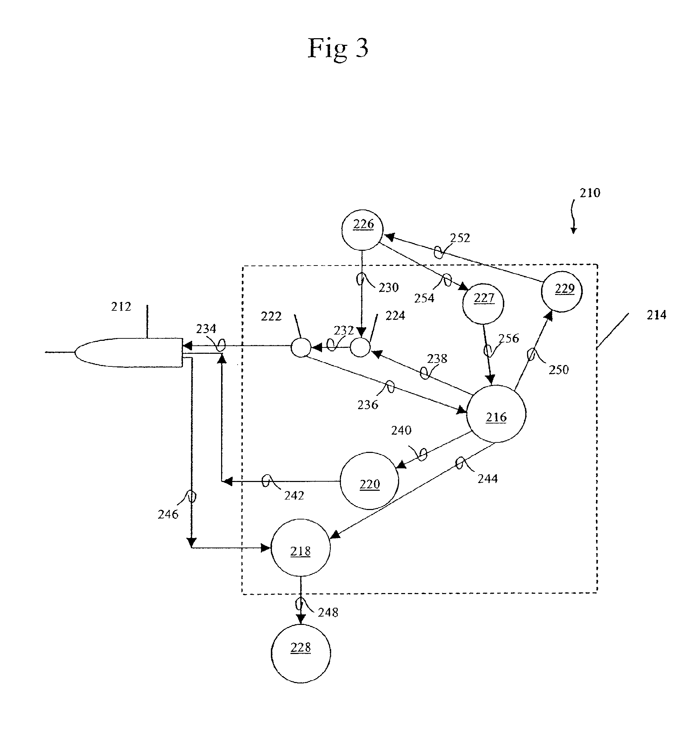

As seen in FIG. 3, control system 210 for use in operating handpiece 212 includes control console 214. Control console 214 generally includes control module or CPU 216, aspiration pump 218, handpiece power supply 220, valve 224, pressurizing source 229, and pressure sensor 227. Flow sensor 222 is connected to handpiece 212 and infusion fluid source 226 through irrigation lines 230, 232 and 234. Infusion source 226 may be any commercially available irrigation solution provided in bottles. Pressurizing source 229 pressurizes infusion fluid source 226 through line 252 and is controlled by CPU 216 through cable 250. Pressurizing source 229 may be any commercially available pressure controller, such as incorporated in the ACCURUS® surgical system available from Alcon Laboratories, Inc., Fort Worth, Texas. Pressure sensor 227 measures the pressure of infusion fluid source 226 through lines 254 and is monitored by CPU 216 through cable 256. Pressure sensor 227 may be any suitable commercially available pressure sensor, such as Model MPX5100 available from Motorola, Inc., Phoenix, Arizona.

In use, sensor 222 measures the flow of irrigation fluid from source 226 to handpiece 212 and supplies this information to CPU 216 through cable 236. The irrigation fluid flow data may be used by CPU 216 to control the operating parameters of console 214 using software commands that are well known in the art. For example, CPU 216, through cable 250, may control pressurizing source 229 while reading pressure sensor 227 data through cable 256 so as to vary the pressure and amount of irrigation fluid reaching handpiece 212 from source 226. CPU 216 may also, through cable 240, vary the output of power supply 220 being sent to handpiece 212 through power cable 242. CPU 216 may also use data supplied by sensor 222 to vary the operation of pump 218 through line 244, which aspirates fluid from handpiece 212 through line 246 and into collection container 228 through line 248. CPU 216 may also use data supplied by sensor 222 and the applied output of power supply 220 to provide audible tones to the user.

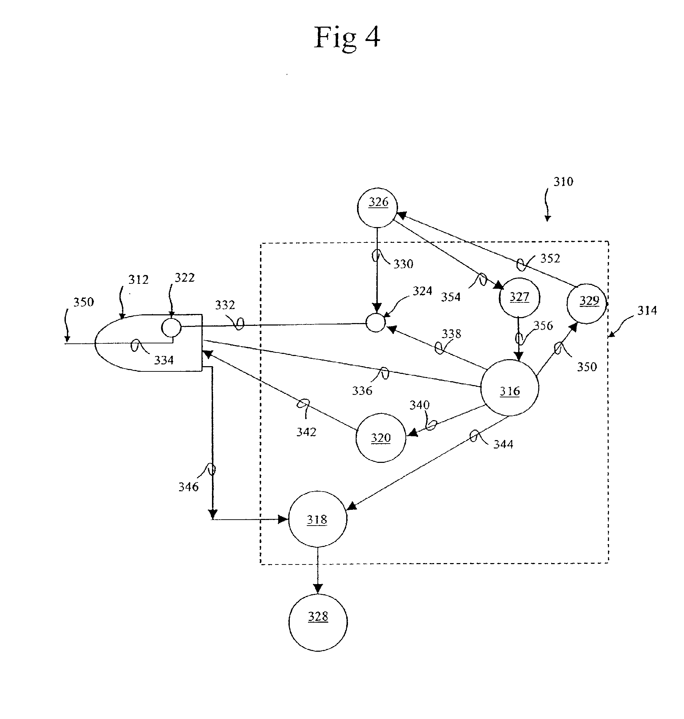

As seen in FIG. 4, control system 310 for use in operating handpiece 312 includes control console 314. Control console 314 generally includes control module or CPU 316, aspiration pump 318, handpiece power supply 320, valve 324, pressurizing source 329, and pressure sensor 327. Flow sensor 322 is contained within handpiece 312. Infusion source 326 may be any commercially available irrigation solution provided in bottles. Pressurizing source 329 may be any commercially available pressure controller. Pressure sensor 327 may be any suitable commercially available pressure sensor.

In use, sensor 322 measures the flow of irrigation fluid from source 326 to handpiece 312 and supplies this information to CPU 316 through cable 336. The irrigation fluid flow data may be used by CPU 316 to control the operating parameters of console 314 using software commands that are well known in the art. For example, CPU 316, through cable 350, may control pressurizing source 329 while reading pressure sensor 327 data through cable 356 so as to vary the pressure and amount of irrigation fluid reaching handpiece 312 from source 326. CPU 316 may also, through cable 340, vary the output of power supply 320 being sent to handpiece 312 through power cable 342. CPU 316 may also use data supplied by sensor 322 to vary the operation of pump 318 through cable 344, which aspirates fluid from handpiece 312 through line 346 and into collection container 328 through line 348. CPU 316 may also use data supplied by sensor 322 and the applied output of power supply 320 to provide audible tones to the user.

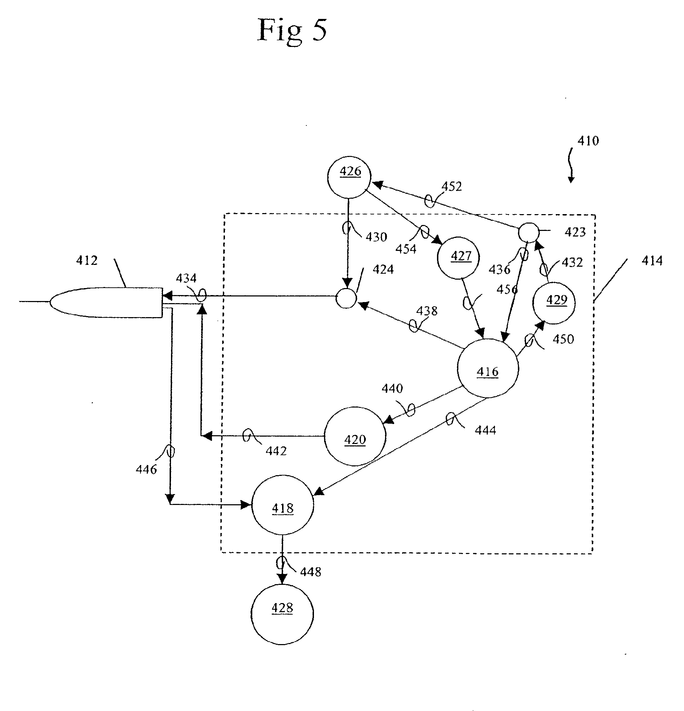

As seen in FIG. 5, control system 410 for use in operating handpiece 412 includes control console 414. Control console 414 generally includes control module or CPU 416, aspiration pump 418, handpiece power supply 420, valve 424, pressurizing source 429, and pressure sensor 527. Airflow sensor 423 is connected to pressurizing source 429 and infusion fluid source 426 through lines 432 and 452. Sensor 423 may be any commercially available flow sensor, such as Model AWM3100V available from Honeywell Micro Switch, Freeport, Illinois. Infusion source 426 may be any commercially available irrigation solution provided in bottles.

In use, sensor 423 measures the flow of air into the infusion fluid source 426 and supplies this information to CPU 416 through cable 436. The airflow data may be used by CPU 416 along with information from pressure sensor 427 for the calculation of infusion flow to the handpiece through line 434. This infusion flow calculation may be used to control the operating parameters of console 414 using software commands that are well known in the art. For example, CPU 416, through cable 450, may control pressurizing source 429 while reading pressure sensor 427 data through cable 456 so as to vary the pressure and amount of irrigation fluid reaching handpiece 412 from source 426. CPU 416 may also, through cable 440, vary the output of power supply 420 being sent to handpiece 412 through power cable 442. CPU 416 may also use this infusion flow calculation to vary the operation of pump 418 through cable 444, which aspirates fluid from handpiece 412 through power cable 442. CPU 416 may also use this infusion flow calculation to vary the operation of pump 418 through cable 444, which aspirates fluid from handpiece 412 through line 446 and into collection container 428 through line 448. CPU 416 may also use this infusion flow calculation and the applied output of power supply 420 to provide audible tones to the user.

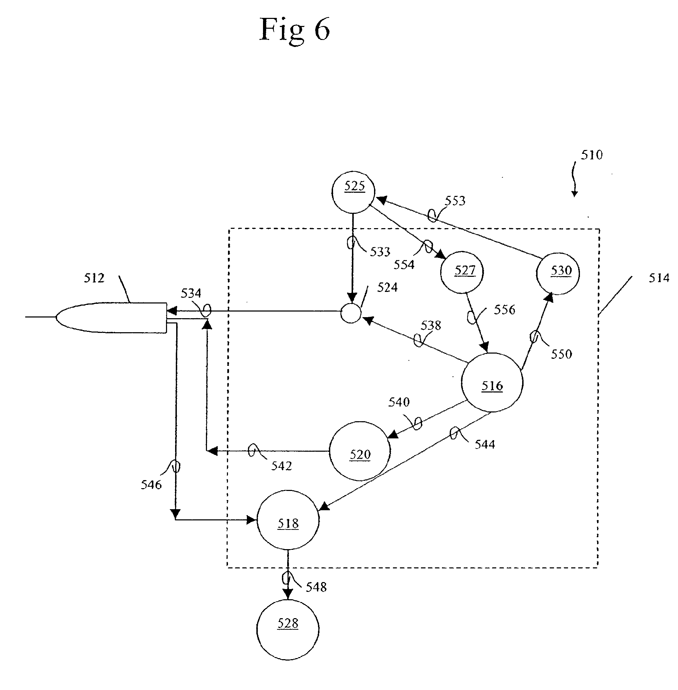

As seen in FIG. 6, control system 510 for use in operating handpiece 512 includes control console 514. Control console 514 generally includes control module or CPU 516, aspiration pump 518, handpiece power supply 520, valve 524, pressurizing source 530, and pressure sensor 527. Infusion source 525 may be any commercially available irrigation solution provided in bags or a custom compliant container. Pressurizing source 530 is a compressing device that squeezes infusion fluid source 525 through mechanism 553 in order to pressurize the fluid. The rate of compression of the infusion fluid source is controlled by CPU 516 through cable 550.

In use, CPU 516 calculates the infusion flow to the handpiece through line 534 based on the compression rate of pressurizing source 530 and the pressure data from pressure sensor 527, it being understood that pressure sensor 527 may communicate directly with infusion fluid source 525 or communicate with infusion fluid source 525 through irrigation lines 533 or 534. This infusion flow calculation may be used to control the operating parameters of console 514 using software commands that are well known in the art. For example, CPU 516, through cable 550, may control pressurizing source 530 while reading pressure sensor 527 data through cable 556 so as to vary the pressure and amount of irrigation fluid reaching handpiece 512 from source 525. CPU 516 may also, through cable 540, vary the output of power supply 520 being sent to handpiece 512 through power cable 542. CPU 516 may also use this infusion flow calculation to vary the operation of pump 518 through cable 544, which aspirates fluid from handpiece 512 through line 546 and into collection container 528 through line 548. CPU 516 may also use this infusion flow calculation and the applied out put of power supply 520 to provide audible tones to the user.

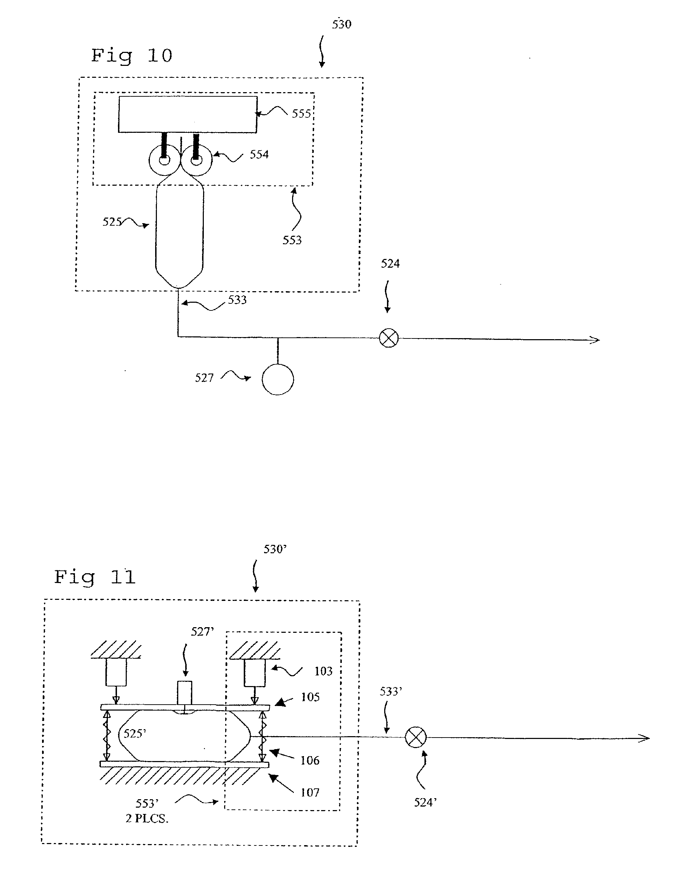

As seen in FIG. 10, pressurizing source 530 includes compression roller mechanism 553, infusion container 525, pressure sensor 527 and infusion or irrigation valve 524. Roller mechanism 553 includes compression rollers 554 and rollers driving motor 555. Infusion container 525 may be a compliant bag such as commonly supplied by Charter Medical, Lakewood, New Jersey, for surgical site infusion or a custom container specifically designed for this application. Infusion container 525 may be made from any suitable material that provides container collapse without excessive stretching. Infusion container 525 may be a thin wall bottle with or without corrugated sides (not shown). Pressure sensor 527 may be any commercially available, disposable, pressure sensor such as Model 1290C manufactured by Hewlett Packard or a custom type sensor specifically made for this application. Infusion valve 524 can be any commercially available pinch type valve commonly used in surgical instruments. Compression roller mechanism 553 may contain bi-directional mechanical rollers 554 and suitable fixturing specifically designed to compress compliant container 525 in a controlled and uniform manner such that the rate of compression is proportional to the rate of fluid expulsion.

In use, compliant container 525 is placed in roller mechanism 553 and connected to irrigation line 533. Infusion valve 524 is opened and the roller mechanism 553 moves to compress container 525. The movement of the rollers mechanism 554 reduces the available volume in container 525, which forces the infusion liquid into irrigation line 533. Information from pressure sensor 527 indicates the infusion pressure and roller mechanism 553 is controlled such that a predetermined infusion pressure reading is maintained. The rate of movement of driving motor 555 is proportional to the rate of liquid expulsion and the information may be used by control system 510 for further systematic control.

As seen in FIG. 11, pressurizing source 530' includes infusion container 525', mechanism 553' pressure sensor 527' and valve 524'. Mechanism 553' includes compression actuators 103, upper plate 105, lower plate 107, and plate return springs 106. Compression actuators 103 may be either worm gear or hydraulically driven and designed to compress plate 105 in a controlled and uniform manner such that the rate of compression is proportional to the rate of fluid expulsion out of container 525'. Return springs 106 can be any commercially available springs used to return the plate to a previous position.

In use, compliant container 525' is placed beneath upper plate 105 and lower plate 107 and connected to irrigation line 533. Infusion valve 524' is opened and actuators 103 operated so as to place downward pressure on plate 105 against springs 106. Downward pressure on plate 105 squeezes container 525' between upper plate 105 and lower plate 107, thereby reducing the available volume in container 525', which forces the infusion liquid into irrigation line 533'. Information from pressure sensor 527' indicates the infusion pressure and actuators 103 are controlled such that a predetermined infusion pressure reading is maintained. The rate of movement of actuators 103 is proportional to the rate of liquid expulsion and the information may be used by control system 510 for further systematic control.

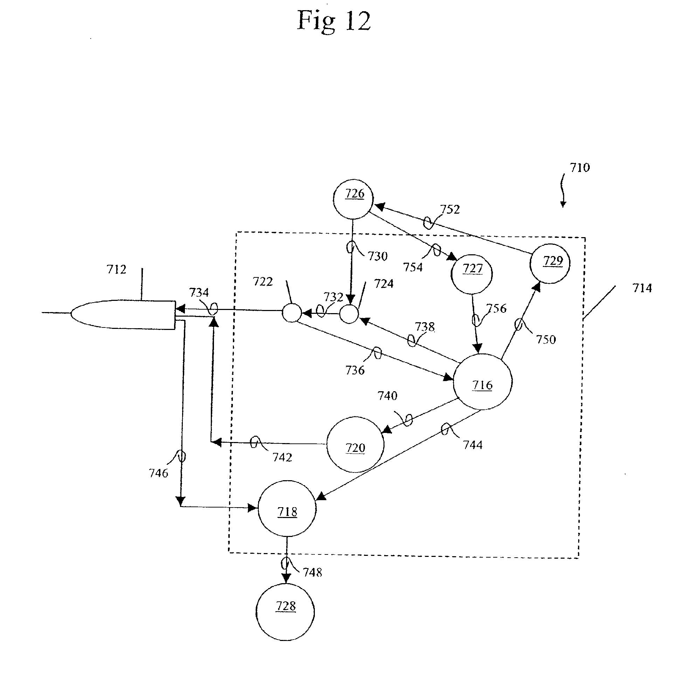

As seen in FIG. 12, in an embodiment of the present invention, control system 710 for use in operating handpiece 712 includes control console 714. Control console 714 generally includes control module or CPU 716, aspiration pump 718, handpiece power supply 720, valve 724, pressurizing source 729, and infusion fluid source pressure sensor 727. Irrigation line pressure sensor 722 is connected to handpiece 712 and infusion fluid source 726 through irrigation lines 730, 732 and 734. Infusion source 726 may be any commercially available irrigation solution provided in bottles. Pressurizing source 729 pressurizes infusion fluid source 726 through line 752 and is controlled by CPU 716 through cable 750. Pressurizing source 729 may be any commercially available pressure controller, such as incorporated in the ACCURUS® surgical system available from Alcon Laboratories, Inc., Fort Worth, Texas. Pressurizing source 729 may also be similar to pressurizing source 530 or 530' described in FIGS. 6, 10 and 11. Pressure sensor 727 measures the pressure of infusion fluid source 726 through lines 754 and is monitored by CPU 716 through cable 756. Pressure sensors 722 and 727 may be any suitable commercially available pressure sensor, such as Model MPX5100 available from Motorola, Inc., Phoenix, Arizona. One skilled in the art will recognize that airflow sensor 423 may be used in addition to or in place of sensor 727, and that the pressure within infusion fluid source 726 can be derived from the operation of pressurizing source 530 or 530'.

In use, sensor 722 measures the pressure of the irrigation fluid in irrigation line 734 at, or even within, handpiece 712 and supplies this information to CPU 716 through cable 736. The irrigation line pressure data may be used by CPU 716 to control the operating parameters of console 714, as described below, using software commands that are well known in the art. For example, CPU 716, through cable 750, may control pressurizing source 729 while reading pressure sensor 727 data through cable 756 so as to vary the pressure and amount of irrigation fluid reaching handpiece 712 from source 726. CPU 716 may also, through cable 740, vary the output of power supply 720 being sent to handpiece 712 through power cable 742. CPU 716 may also use data supplied by sensor 722 to vary the operation of pump 718 through line 744, which aspirates fluid from handpiece 712 through line 746 and into collection container 728 through line 748. CPU 716 may also use data supplied by sensor 722 and the applied output of power supply 720 to provide audible tones to the user.

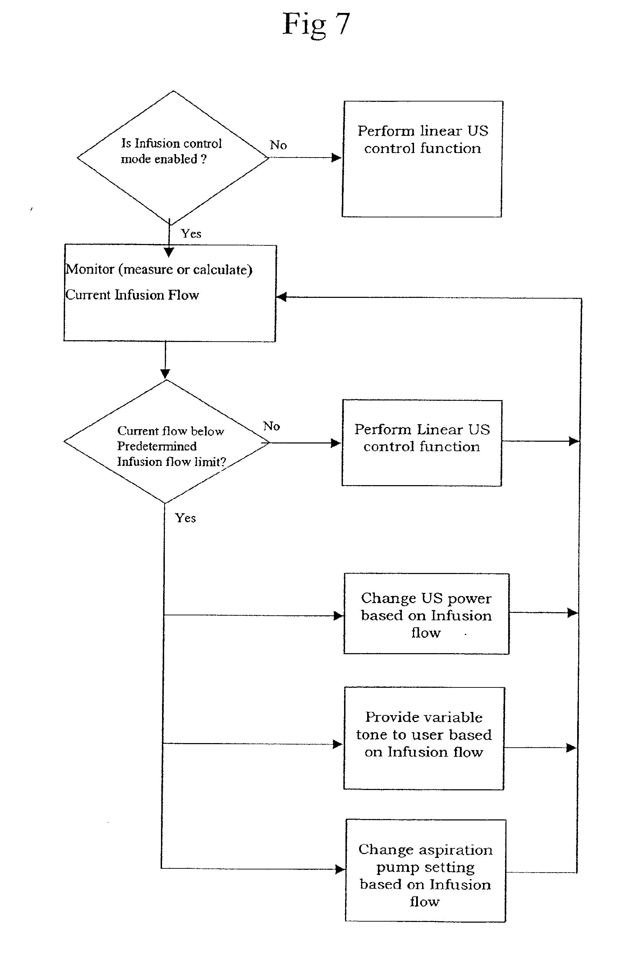

As seen in FIG. 7, when the system of the present invention is monitoring infusion flow, the system monitors the current infusion flow and compares the actual flow against a predetermined flow rate. If infusion flow is above the predetermined rate, no action is taken by the system. If the infusion flow is below the predetermined rate, the system may take a variety of actions, such as changing the power delivered to the ultrasound handpiece, providing a variable tone to the surgeon or changing the aspiration pressure.

The system of the present invention may be used to control intraoperative intraocular pressure (IOP) and/or temperature at the wound. To control IOP, the user sets a desired IOP or IOP range (IOPₛₑₜ) in CPU 716. Infusion fluid line pressure information from sensor 722 (Pᵢᵣᵣ) is provided to CPU 716, and infusion fluid source pressure information from sensor 727 is provided to CPU 716 (P_{bot}). CPU 716 determines the flow out of handpiece 712 using the equation Flow = (P_{bot} - Pᵢᵣᵣ)/ R_{bot} where R_{bot} is the fludics resistance between sensor 727 and sensor 722. CPU 716 computes the actual IOP within the surgical site (IOP_{act}) using the equation IOP_{act} = Pᵢᵣᵣ - (Flow * Rᵢᵣᵣ) where Rᵢᵣᵣ is the fluidics resistance between sensor 722 and the surgical site. CPU compares IOP_{act} to IOPₛₑₜ and adjusts pressurizing source 729 accordingly to maintain IOPₛₑₜ. The logic process in CPU 716 can be programmed in a variety of ways, such as PID algorithm, fuzzy logic algorithm or any other suitable algorithm. Alternatively, if pressurizing source 530 or 530' is used, the flow out of handpiece 712 will be proportional to the rate of compression of infusion fluid source 726, so pressurizing source 530 or 530' may be used to monitor infusion flow out of handpiece 712 and sensor 722 is not required.

The system of the present invention may also be used to control temperature at the wound site. To control wound site temperature, the user may select a wound site temperature (Tempₘₐₓ) and desired action in CPU 16, 116, 216, 316 or 416. Alternatively, the user may select a desired temperature range. Infusion fluid flow information from sensor 22, 122, 222, 322 or 423 is provided to CPU 16, 116, 216, 316 or 416, respectively, and to CPU 16, 116, 216, 316 or 416 calculates current wound site temperature (Temp_{cur}) based on current and previous values of infusion fluid flow and ultrasound power. When Temp_{cur} approaches Tempₘₐₓ, for example, the user may desire for the ultrasound power to be decreased, the duty cycle or amplitude of the ultrasound power to be varied, the infusion pressure and/or infusion fluid flow to be varied the aspiration pressure to be varied, or for an audible tone to be sounded. The user could program a variety of actions to take place depending upon the value selected for Tempₘₐₓ or the rate at which Temp_{cur} approaches Tempₘₐₓ. The logic process in CPU 16, 116, 216, 316 or 416 can be programmed in a variety of ways, such as PID algorithm, fuzzy logic algorithm or any other suitable algorithm.

This description is given for purposes of illustration and explanation. It will be apparent to those skilled in the relevant art that changes and modifications may be made to the invention described above without departing from its scope.

## Claims

1. Apparatus for controlling an intraoperative temperature at a wound site, comprising a control system having,
i) a control console (14,114,214,314,414,514,714) having a control module including the central processing unit (16,116,216,316,416,516,716);
ii) a handpiece power supply (20,120,220,320,420,520,720) and
iii) a means for measuring infusion fluid flow (22,122,222,322,422,522,722);
and **characterized by**;
a. means for selecting a desired intraoperative wound site temperature;
b. means for providing infusion fluid flow information to the control module;
c. means for providing handpiece power information to the control module from the handpiece power supply;
d. means for calculating a current temperature at the would site based on current and previous values of infusion fluid flow and handpiece power;
e. means for comparing the calculated current temperature at the wound site with the selected desired intraoperative wound site temperature; and
f. means for varying the operation of the handpiece power supply based on the comparison between the calculated current temperature at the wound site with the selected desired intraoperative wound site temperature.

2. Apparatus according to claim 1, wherein the control console further includes an aspiration pump (18,118,218,318,418,518,718) and the control module is capable of varying the operation of the aspiration pump based on the calculated current wound site temperature.

3. Apparatus according to claim 1, wherein the control console is capable of providing audible tones based on the calculated current wound site temperature.

4. Apparatus according to claim 1, wherein the control console further includes an infusion fluid source (26,126,226,326,426,526,726) and a pressurizing source (530,530',729) for the infusion fluid source.

5. Apparatus according to claim 1, wherein the infusion fluid source (26,126,226,326,426,526,726) is flexible and the pressurizing source (530,530',729) includes a mechanism (554, 553') to compress the infusion fluid source.

6. Apparatus according to claim 1, wherein the means for measuring infusion flow (22,122,222,322,422,522,722) comprises a mechanism to compress the infusion fluid source.

7. Apparatus according to claim 5 or claim 6, wherein the mechanism (554, 553') to compress the infusion fluid source comprises a roller mechanism (554) or a compression plate (553').

8. Software which when executing on a central processing unit is configured to perform the operation of a central processing unit (16,116,216,316,416,516,716) in a control system for a surgical device having a handpiece (12,112, 212, 312, 412, 512,712) with a handpiece power supply (20,120,220,320,420,520,720), adapted to deliver infusion fluid from a source to, and draw aspiration fluid from, an operative site, said control system comprising a control console (14,114,214,314,414,514,714) having a control module including the central processing unit, and a means for measuring infusion flow (22,122,222,322,422,522,722) in the control console or in the handpiece, capable of providing infusion fluid flow data to the central processing unit,
**characterized by** software-implemented steps of;
a. selecting a desired intraoperative wound site temperature;
b. providing infusion fluid flow information to the control module;
c. providing handpiece power information to the control module from the handpiece power supply;
d. calculating a current temperature at the would site based on current and previous values of infusion fluid flow and handpiece power;
e. comparing the calculated current temperature at the wound site with the selected desired intraoperative wound site temperature; and
f. varying the operation of the handpiece power supply based on the comparison between the calculated current temperature at the wound site with the selected desired intraoperative wound site temperature.

9. Software according claim 8, further comprising the step of varying the operation of an aspiration pump based on the calculated current wound site temperature.

10. Software according claim 8, further comprising the step of providing audible tones based on the calculated current wound site temperature.
